# EUROPEAN PATENT APPLICATION

(11) **EP 2 377 947 A1**
(43) Date of publication of application: **19.10.2011**
(21) Application number: 09833417.0
(22) Date of filing: 14.12.2009
(51) Int. Cl.: C12Q 1/04, G01N 33/48, C12N 15/09

(54) **METHOD FOR DETECTING CANCER CELLS IN BLOOD SAMPLE**

(30) Priority: 18.12.2008 JP 2008322439
(71) Applicant: Sysmex Corporation, Kobe-shi, Hyogo 651-0073 (JP)
(72) Inventor: SATO, Jun, Kobe-shi Hyogo 651-0073 (JP); MASAGO, Akinori, Kobe-shi Hyogo 651-0073 (JP); TSUJINO, Yukio, Kobe-shi Hyogo 651-0073 (JP)
(74) Representative: Paemen, Liesbet R.J.
(86) International application number: PCT/JP2009/070843
(87) International publication number: WO 2010/071114

(57) **Abstract**

A method for detecting cancer cells in a blood sample is provided comprising the steps of proliferating an oncolytic virus at least in the cancer cells by incubating the blood sample being suspected to contain the cancer cells with the oncolytic virus; mixing the blood sample obtained from the proliferating step with a fixing agent and a nonionic surfactant; and detecting the cancer cells in the blood sample obtained from the mixing step, in which the oncolytic virus has been proliferated.

SEQUENCE LISTING

<110>  Sysmex Corporation

<120>  Method for detecting cancer cells in blood sample

<130>  TM5377PC

<150>  JP2008-322439
<151>  2008-12-18

<160>  1

<170>  PatentIn version 3.1

<210>  1
<211>  455
<212>  DNA
<213>  Homo sapiens

<400>  1
tggcccctcc ctcgggttac cccacagcct aggccgattc gacctctctc cgctggggcc      60

ctcgctggcg tccctgcacc ctgggagcgc gagcggcgcg cgggcgggga agcgcggccc     120

agacccccgg gtccgcccgg agcagctgcg ctgtcggggc caggccgggc tcccagtgga     180

ttcgcgggca cagacgccca ggaccgcgct ccccacgtgg cggagggact ggggacccgg     240

gcacccgtcc tgccccttca ccttccagct ccgcctcctc cgcgcggacc ccgccccgtc     300

ccgacccctc ccgggtcccc ggcccagccc cctccgggcc ctcccagccc ctccccttcc     360

tttccgcggc cccgccctct cctcgcggcg cgagtttcag gcagcgctgc gtcctgctgc     420

gcacgtggga agccctggcc ccggccaccc ccgcg                                455

## Description

### TECHNICAL FIELD

The present invention relates to a method for detecting cancer cells in blood samples. The present invention also relates to a reagent kit that can be used for the method.

### BACKGROUND ART

It is believed that cancer metastasis develops when cancer cells from a primary focus spread systemically through blood vessels and lymphatic vessels and then some of the cells are engrafted to and proliferate in organs in other parts. Cancer cells circulating in blood are called CTCs (Circulating Tumor Cells). It has been reported that the number of CTCs correlates with cancer metastasis and prognosis, so that it is believed that counting the number of CTCs is useful in, for example, predicting prognoses and treatment efficacy of metastatic cancer such as metastatic breast cancer.

The conventional CTC detecting methods include a method in which cancer cells in blood are captured with antibodies directed against the cancer cells and the captured cancer cells are labeled and detected with fluorescent-labeled antibodies (see Japanese Unexamined Patent Publication No. 2007-178193 (Patent Literature 1) and Japanese Translation of PCT International Application No. 2002-503814 (Patent Literature 2)).

Other known techniques include the one which utilizes the fact that cancer cells have increased telomerase activity while the most of normal cells have undetectable telomerase activity. More specifically, according to this technique, a virus (Oncolytic Virus) comprising a replication cassette containing a telomerase promoter and a label-expressing cassette containing a label protein (e.g. Green Fluorescent protein (GFP)) is proliferated in cancer cells to specifically label cancer cells (see Japanese Unexamined Patent Publication No. 2004-33186 (Patent Literature 3) and WO 2006/36004 (Patent Literature 4)).
Oncolytic viruses containing a GFP gene have been on the market in the name of Telomescan^{®} (OBP-401). Telomescan^{®} can proliferate specifically in cancer cells and produce GFP to allow cancer cells be specifically fluorescent.

### Citation List

### Patent Literature

Patent Literature 1: Japanese Unexamined Patent Publication No. 2007-178193
Patent Literature 2: Japanese Translation of PCT International Application No. 2002-503814
Patent Literature 3: Japanese Unexamined Patent Publication No. 2004-33186
Patent Literature 4: WO 2006/36004

### SUMMARY OF INVENTION

### Technical Problem

Oncolytic viruses such as Telomescan^{®} have low infection and proliferation rates in normal blood cells and can specifically proliferate in "living" cancer cells. Thus, it is believed that "living" cancer cells in blood, i.e. CTCs can be detected with oncolytic viruses.

However, the following problems need to be solved in order to establish a system for detecting cancer cells in blood with oncolytic viruses:
- Removal of erythrocytes: the number of cancer cells in blood is extremely low. Thus, there is a possibility that cancer cells are masked by numerous erythrocytes in blood, and hence the detection of cancer cells is interfered.
- Suppression of false-positive signals: on rare occasions, oncolytic viruses can proliferate in normal blood cells such as leukocytes (some monocytes and lymphocytes). Such proliferation of oncolytic viruses in normal cells may give false-positive signals in detection of cancer cells.

### Solution to Problem

The present inventors have made an extensive study in order to solve the problems and found that when blood samples in which oncolytic viruses have been proliferated are treated with a fixing agent and a nonionic surfactant, erythrocytes are removed as well as false-positive signals from normal cells such as leukocytes can be reduced, thus accomplishing the present invention.

Thus, the present invention provides a method for detecting cancer cells in a blood sample comprising the steps of:
proliferating an oncolytic virus at least in the cancer cells by incubating the blood sample being suspected to contain the cancer cells with the oncolytic virus;
mixing the blood sample obtained from the proliferating step with a fixing agent and a nonionic surfactant; and
detecting the cancer cells in the blood sample obtained from the mixing step, in which the oncolytic virus has been proliferated.

The present invention also provides a reagent kit for detection of cancer cells in a blood sample comprising a first reagent containing an oncolytic virus, a second reagent containing a fixing agent and a third reagent containing a nonionic surfactant.

### Advantageous Effects of Invention

The present method and reagent kit allow convenient, sensitive and accurate detection of a minute amount of cancer cells, i.e. CTCs, in blood samples.

### BRIEF DESCRIPTION OF DRAWINGS

Fig. 1 is a graph of fluorescent intensities measured in Examples 1 to 3 and Comparative Example 1.
Fig. 2(A) shows a micrograph obtained in Example 4 when Emulgen 2025G was used as a surfactant. "WBC" in figures is an abbreviation for leukocytes.
Fig. 2(B) shows a micrograph obtained in Example 4 when Nikkol BL-9EX was used as a surfactant.
Fig. 2(C) shows a micrograph obtained in Example 4 when Nikkol BO-20V was used as a surfactant.
Fig. 2(D) shows a micrograph obtained in Example 4 when Nikkol BT-12 was used as a surfactant.
Fig. 2(E) shows a micrograph obtained in Example 4 when Emulgen 2020G was used as a surfactant.
Fig. 2(F) shows a micrograph obtained in Example 4 when Nissan Cation AB600 was used as a surfactant.
Fig. 2(G) shows a micrograph obtained in Example 4 when Nissan Cation BB was used as a surfactant. "RBC" in figures is an abbreviation for erythrocytes.
Fig. 2(H) shows a micrograph obtained in Comparative Example 2.

### DESCRIPTION OF EMBODIMENTS OF THE INVENTION

According to the present invention, cancer cells in blood samples (CTCs) may be any cancer cells which have telomerase activity and include not only cancer cells which have been dropped from solid cancers and entered into peripheral blood but also hematologic cancers. CTCs detected with the method and reagent kit according to the present embodiment are, but not limited to, preferably CTCs derived from solid cancers, more preferably CTCs derived from breast cancers.

### <Oncolytic viruses>

As used herein, "oncolytic virus" refers to a conditionally replicative virus which is specifically proliferative in living cancer cells but is not normally proliferative in normal cells.
However, it should be clearly understood by a person skilled in the art that due to a mechanism to render it conditionally replicative, oncolytic viruses may proliferate in normal cells.
Namely, oncolytic viruses can specifically proliferate in cancer cells due to a mechanism that renders them specifically proliferative in cancer cells such as a promoter that specifically shows promoter activity in cancer cells; some normal cells may cause the activation of such a promoter (e.g. leukocytes). In methods for detecting cancer cells in which cells are detected as cancer cells in which the oncolytic virus has been proliferated, such normal cells may be detected as cancer cells, leading to false-positive results.

However, according to the method of the present invention, normal cells are selectively damaged without affecting the cancer cells in which the oncolytic virus has been proliferated by treatment with the fixing agent and the nonionic surfactant. Therefore, false-positive signals can be decreased which may be generated from the normal cells.
Further, according to the method of the present invention, erythrocytes in blood samples can also be lysed by the above treatment. Therefore, cancer cells which may exist in a minute amount in blood samples can be detected with higher sensitivity.

As used herein, "to damage cell(s)" means to destroy at least a part of the cell membrane of the cell(s) to release intracellular components.
As used herein, "to lyse erythrocyte(s)" means to solubilize the cell membrane of erythrocyte(s).

The oncolytic virus includes, but is not limited to, viruses in which a promoter is incorporated which specifically shows promoter activity in cancer cells. Promoters which specifically show promoter activity in cancer cells (hereinafter referred to as "cancer cell specific promoters") include the human telomerase promoter, the human prostate-specific antigen (PSA) promoter, the human alpha-fetoprotein (AFP) promoter, the carcinoembryonic antigen (CEA) promoter and the like. The human telomerase promoter is preferred because it can show promoter activity in various types of cancer cells. Among others, the hTERT promoter is preferred which is the gene encoding human telomerase reverse transcriptase.

The nucleic acid sequence of the hTERT promoter is shown in SEQ ID NO:1. The hTERT promoter functions with its whole nucleic acid sequence of 455 bp shown in SEQ ID NO:1. As it is believed that the 181 bp core region in its 5' upstream area is important for the downstream gene expression, it is sufficient to use nucleic acid sequences comprising this core region.

Oncolytic virus preferably expresses a label protein. Thus, the oncolytic virus preferably contains a label protein-encoding gene. As used herein, "a label protein" means a detectable protein which allows, upon its expression in cells, differentiation of the cells from other cells in which the protein is not expressed. The label protein may be label proteins conventionally used in the biochemical field and may include fluorescent proteins such as the green fluorescent protein (GFP) and its mutant (e.g. enhanced-humanized GFP (EGFP), red-shift GFP (rsGFP)), the yellow fluorescent protein (YFP) and the blue fluorescent protein (BFP).
The label protein is preferably the green fluorescent protein.

The label protein gene may be placed under control of the cancer cell specific promoter. Alternatively, the label protein gene may be placed under control of a promoter which shows promoter activity in the oncolytic virus.
The promoter which can control expression of the label protein gene includes the cytomegalovirus (CMV) promoter, the SV40 late promoter, the MMTV LTR promoter, the RSV LTR promoter, the Sr promoter and the like. Among others, the label protein gene is preferably placed under control of the CMV promoter or the hTERT promoter. The nucleic acid sequences of these promoters are well-known.

An expression cassette which comprises at least a gene encoding the label protein and a promoter for expression of the label protein (a cancer cell specific promoter or a promoter which may show promoter activity in the oncolytic virus) can be obtained according to conventional gene engineering techniques. For example, the expression cassette can be obtained by amplifying the label protein-encoding gene and the promoter by, for example, polymerase chain reaction (PCR) according to known sequences, ligating the amplification products of the genes to a suitable plasmid and excising a desired region(s) (see, for example, WO 2006/36004).

The oncolytic virus may contain a replication cassette in which a gene(s) necessary for the proliferation of the virus is(are) linked downstream of the cancer cell specific promoter. The genes necessary for the proliferation of the virus include early genes (E1A, E1B, etc.), a protein production initiation signal specific for *Picornaviridae,* IRES and the like. The nucleic acid sequences of these genes are well-known.

The replication cassette can be prepared according to conventional gene engineering techniques. For example, the replication cassette can be obtained by amplifying the cancer cell specific promoter and optionally a gene necessary for the proliferation of the virus by PCR according to the well-known sequences, ligating the amplification products of the genes to a suitable plasmid and excising a desired region(s) (see, for example, WO 2006/36004).

The above virus may be derived from adenovirus, herpes simplex virus, Sendai virus or reovirus. Among others, human adenovirus is preferred.

The oncolytic virus may be the one commercially available. The oncolytic virus is preferably Telomescan^{®} (OBP-401) (Oncolys Biopharma Inc.), which is the adenovirus having a replication cassette containing the hTERT promoter, the E1A gene, the IRES gene and the E1B gene and an expression cassette containing the CMV promoter and the GFP gene.

### <Proliferating step>

The method of the present invention comprises the step of proliferating the oncolytic virus at least in the cancer cells by incubating a blood sample being suspected to contain the cancer cells with the oncolytic virus (hereinafter also referred to as "the proliferating step").
The above blood samples may be generally obtained from subjects from which the presence of cancer cells are to be detected, such as patients who are suspected to be suffering from cancer or patients having cancer. The blood samples may be either of whole blood and processed blood which is obtained by pre-treating whole blood. The blood samples are preferably whole blood and especially peripheral blood from which serum has been deprived because of the improved detection efficiency.
Serum can be deprived from whole blood with well-known methods such as centrifuging whole blood to which an anti-coagulant (e.g. ethylenediaminetetraacetic acid, sodium citrate, heparin etc.) has been added. The centrifugation is preferably carried out at 500 to 3500 rpm for 3 to 30 minutes.

The incubation of a blood sample and the oncolytic virus is preferably carried out in the presence of a conventional medium for animal cell cultures. Such medium includes RPMI-1640, Dulbecco's modified Eagle's medium (DMEM), minimum essential medium (MEM) and the like.

The amount of the oncolytic virus to be incubated with a blood sample is preferably 6 x 10⁴ to 6 x 10⁸ PFU (Plaque Forming Unit) per ml of the blood sample.
The time period and temperature conditions for the incubation of a blood sample and the oncolytic virus can be appropriately adjusted according to the type of the oncolytic virus as long as the oncolytic virus can proliferate at least in the cancer cells in the blood sample. The incubation conditions are preferably at the temperature of 25 to 40°C, more preferably 30 to 37°C and for 1 to 36 hours, more preferably 12 to 24 hours.

Due to the above incubation, the oncolytic virus can infect cancer cells in a blood sample and proliferate in the cells.
However, leukocytes (monocytes, lymphocytes etc.) occasionally express enzymes such as telomerase which cancer cells specifically express. Thus, due to the incubation as above, the oncolytic virus may proliferate in those normal leukocytes (i.e. non-cancer cells).

### <Mixing step>

The method of the present invention comprises the step of mixing the blood sample obtained from the proliferating step with a fixing agent and a nonionic surfactant (hereinafter also referred to as "the mixing step").
In the mixing step, a blood sample may be mixed with the nonionic surfactant followed by mixing of the fixing agent, or a blood sample may be mixed with the fixing agent followed by mixing of the nonionic surfactant, or a blood sample, the fixing agent and the nonionic surfactant may be mixed at the same time. More preferably, a blood sample is mixed with the fixing agent followed by mixing of the nonionic surfactant.

The blood sample may be deprived of fractions other than a cell fraction by a technique such as centrifugation prior to mixing with the fixing agent and the nonionic surfactant.

In the mixing step, a concentration of the nonionic surfactant when it is mixed with a blood sample is preferably a concentration which damages leukocytes without damaging cancer cells. Such concentration may be appropriately selected according to the type of the nonionic surfactant and is preferably 0.003 to 0.4% by weight, more preferably 0.030 to 0.037% by weight.
As used herein, "a concentration of the nonionic surfactant when it is mixed with a blood sample" means not only "a concentration of the nonionic surfactant when it is mixed directly with a blood sample" but also "a concentration of the nonionic surfactant when it is mixed with a mixture of the blood sample and the fixing agent". Namely, when a blood sample and the fixing agent are mixed prior to mixing the nonionic surfactant in the mixing step, the above concentration of the nonionic surfactant is the one in the mixture of the blood sample, the fixing agent and the nonionic surfactant.

The nonionic surfactant may be either of polyoxyethylene surfactants and polyoxy compound fatty acid ester surfactants, among which polyoxyethylene surfactants are preferred. Polyoxyethylene surfactants include higher alcohol-ethylene oxide adducts, alkylphenol-ethylene oxide adducts, higher fatty acid-ethylene oxide adducts, higher aliphatic amine-ethylene oxide adducts, higher fatty acid amide-ethylene oxide adducts and the like. Among others, higher alcohol-ethylene oxide adducts are preferred.

An alkyl group of the higher alcohol of the higher alcohol-ethylene oxide adducts may be linear or branched and has preferably 12 to 30, more preferably 15 to 25 carbon atoms. The higher alcohol-ethylene oxide adducts preferably have a polymerization degree of ethylene oxide of 15 to 40, more preferably 20 to 30.
The specific higher alcohol-ethylene oxide adducts having a branched alkyl group-containing higher alcohol are represented as the following formula (I). 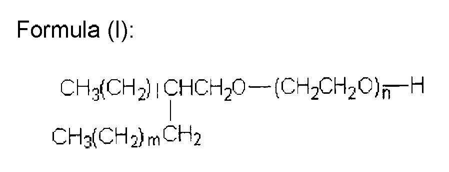 wherein I is an integer of 6 to 24, m is an integer of 1 to 19 and n is an integer of 15 to 40.

The nonionic surfactant is preferably polyoxyethylene octyldodecyl ether which has a branched alkyl group-containing higher alcohol, contains 20 carbon atoms and has a polymerization degree of ethylene oxide of 25.

In the mixing step, a concentration of the fixing agent when it is mixed with a blood sample is preferably a concentration which lyses erythrocytes. Such concentration is preferably 1 to 7% by weight, more preferably 2 to 4% by weight.
As used herein, "a concentration of the fixing agent when it is mixed with a blood sample" means not only "a concentration of the fixing agent when it is mixed directly with a blood sample" but also "a concentration of the fixing agent when it is mixed with a mixture of the blood sample and the nonionic surfactant". Namely, when a blood sample and the nonionic surfactant are mixed prior to mixing the fixing agent, the above concentration of the fixing agent is the one in the mixture of the blood sample, the fixing agent and the nonionic surfactant.

The fixing agent may be a substance which allows crosslinking of proteins and which is usually used for tissue fixation, and preferably is an aldehyde compound such as paraformaldehyde, glutaraldehyde and formaldehyde. Among others, paraformaldehyde is preferred.

The mixing step is preferably carried out under the condition which does not damage cancer cells. Such condition includes pH of 6 to 9 and an osmotic pressure of 200 to 400 mOsm.

### <Detecting step>

The method of the present invention comprises the step of detecting the cancer cells in the blood sample obtained from the mixing step, in which the oncolytic virus has been proliferated (hereinafter also referred to as "the detecting step").
The detecting step is preferably the step of detecting the above described label protein expressed due to the proliferation of oncolytic virus.

The label protein can be appropriately detected according to the methods appropriately selected depending on the type of the label protein. When the label protein is fluorescent proteins, the label protein can be detected by fluorescent microscopy, flow cytometric detection and the like.

The fluorescent microscopy can be carried out by, for example, preparing a smear sample on a slide from the blood sample obtained from the mixing step with a conventional method, observing the smear sample under a fluorescent microscope equipped with a CCD camera, detecting fluorescent cells and optionally counting the cells.

When a flow cytometer is used, the detection can be carried out by, for example, subjecting the blood sample obtained from the mixing step to a flow cytometer capable of detecting fluorescence, detecting fluorescent cells and optionally counting the cells.

### <Additional step(s)>

The method of the present invention can further comprise the step of staining dead cells. By including such a step, "live" cancer cells and "dead" cancer cells can be detected to allow a calculation of viability of cancer cells in blood samples. This step is preferably carried out prior to the step of mixing the blood sample with the fixing agent and the nonionic surfactant.
The step of staining dead cells can be carried out by bringing the blood sample into contact with a reagent specifically staining dead cells. "A reagent specifically staining dead cells" refers to a reagent that can stain dead cells distinctively from living cells. The reagent specifically staining dead cells can be a commercially available kit and is preferably Live/Dead Fixable Red Dead Cell Stain Kit (Invitrogen).

### <Reagent kit>

The present invention also provides a reagent kit for detection of cancer cells in a blood sample. The present reagent kit comprises a first reagent containing the oncolytic virus, a second reagent containing the fixing agent and a third reagent containing the nonionic surfactant.

The first, second and third reagents may be in the form of liquid or in the form of solid that can be reconstituted by adding an appropriate solvent upon use.

The first reagent preferably comprises the oncolytic virus in a concentration such that the oncolytic virus in an amount of 6 x 10⁴ to 6 x 10⁸ PFU per ml of a blood sample is provided when it is incubated with the blood sample in the form of liquid.

The second reagent preferably comprises the fixing agent in an amount such that erythrocytes are lysed when it is mixed with a blood sample. More specifically, the second reagent comprises the fixing agent in an amount such that the fixing agent concentration of preferably 1 to 7% by weight, more preferably 2 to 4% by weight is provided when it is mixed with a blood sample. When a blood sample is first mixed with the third reagent, the second reagent comprises the fixing agent such that the above concentration of the fixing agent is achieved in the mixture of the blood sample, the second reagent and the third reagent.

The third reagent preferably comprises the nonionic surfactant in an amount such that leukocytes are damaged without cancer cells being damaged when it is mixed with a blood sample. More specifically, the third reagent comprises the nonionic surfactant in an amount such that the nonionic surfactant concentration of preferably 0.003 to 0.4% by weight, more preferably 0.030 to 0.037% by weight is provided when it is mixed with a blood sample. When a blood sample is first mixed with the second reagent, the third reagent comprises the nonionic surfactant such that the above concentration of the nonionic surfactant is achieved in the mixture of the blood sample, the second reagent and the third reagent.

The second and third reagents may comprise an appropriate buffer and osmoticum in order to achieve a condition which does not damage cancer cells when they are mixed with a blood sample. The condition which does not damage cancer cells is preferably pH of 6 to 9 and an osmotic pressure of 200 to 400 mOsm. Thus, the buffer may include phosphate buffered saline (PBS), citrate buffer, HEPES and the like. The osmoticum may include ethylene glycol, sodium chloride and the like.

### Examples

The present invention is now more specifically illustrated according to the following Examples. However, it is not intended to limit the present invention by the following Examples.

### 1. Effect of proliferation of oncolytic virus on mononuclear cell fraction

When cancer cells in blood samples are to be detected using an oncolytic virus, the virus may proliferate in mononuclear cells contained in the samples so that the cells are detected as cancer cells.
Accordingly, the mononuclear cell fraction was collected from blood obtained from human subjects and was subjected to treatment according to the present method (treatment with a fixing agent and a nonionic surfactant) to see whether or not the influence by mononuclear cells on the detection can be reduced. "Mononuclear cell" is the general term for monocytes and lymphocytes.

### Example 1

### <Preparation of mononuclear cells>

Vacuum blood collection tubes (Venoject^{®} II Vacuum Blood Collection tube (4.5 ml), containing 3.8% sodium citrate, TERUMO Corporation) were used to collect blood from two healthy female subjects. Blood (5.0 ml) drawn within last two hours was carefully layered in 15-ml centrifuge tubes into which 5.0 ml of Polymorphprep^{™} (Daiichi Pure Chemicals Co., Ltd.), a reagent for separation of mononuclear cells, was placed beforehand. The centrifuge tubes were centrifuged at room temperature and 500 x g for 30 minutes in a swing rotor. The mononuclear cell floating fluid was transferred to new centrifuge tubes with a Pasteur pipette, into which an equivalent volume of PBS (-) was then added and mixed. This mixture was centrifuged at room temperature and 400 x g for 10 minutes and a supernatant was removed to obtain the precipitated mononuclear cells. The obtained mononuclear cells were resuspended into PBS (-), centrifuged at 400 x g for 10 minutes and a supernatant was removed to obtain the mononuclear cell fraction.

### <Virus proliferation>

To the obtained mononuclear cell fraction was added RPMI-1640 to 10 ml. To this was added the oncolytic virus (Telomescan^{®} (OBP-401), Oncolys BioPharma Inc.) to the final concentration of 6 x 10⁶ PFU/ml, and was cultured at 37°C for 24 hours with rotation. The obtained culture was centrifuged at 1500 rpm for 5 minutes with weak break, and then a supernatant was removed to obtain the cells in which oncolytic virus had been proliferated (hereinafter also referred to as "the proliferated cells").

### <Treatment with fixing agent and nonionic surfactant>

To the obtained proliferated cells was added an equivalent volume of 4% paraformaldehyde (PFA) and the mixture was left to stand at room temperature for 20 minutes. A 0.05% by weight solution of nonionic surfactant (Emulgen 2025G, Kao Corporation) in PBS (-) in a two-fold volume of the total volume of the proliferated cells and PFA was added and suspended. The suspension was incubated at 40°C for 5 minutes with stirring to obtain a treated sample. The chemical structure of Emulgen 2025G is shown in the following formula (II). 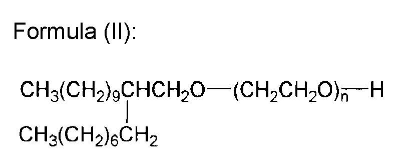 wherein n is 25.

### <Slide preparation and microscopy>

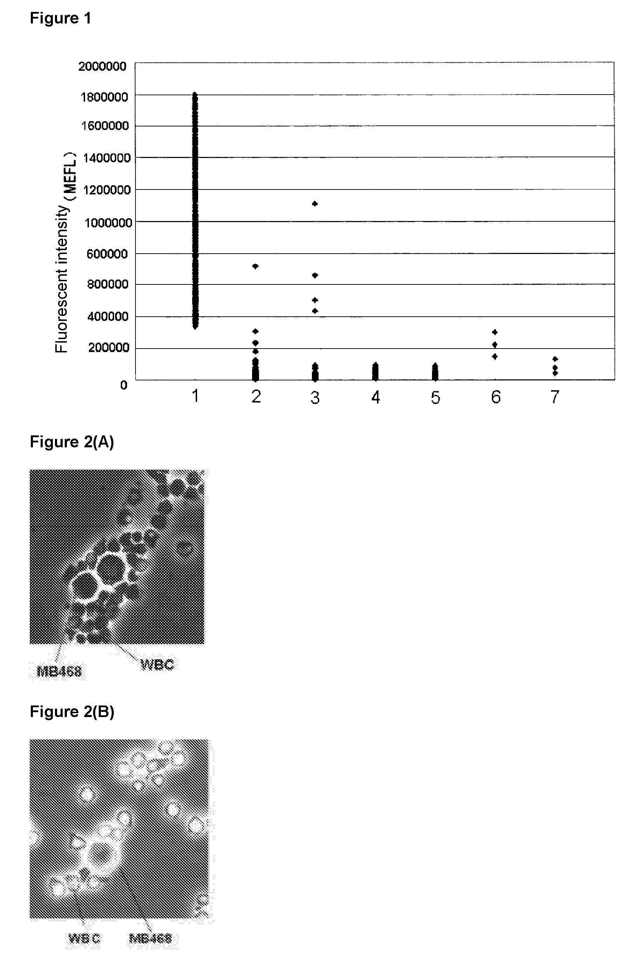

The obtained treated sample was centrifuged at 1500 rpm for 5 minutes with weak break, a supernatant was removed and a precipitate was obtained. The obtained precipitate was suspended in 1 ml PBS. The obtained suspension was centrifuged at 1500 rpm for 5 minutes with weak break, a supernatant was removed and a slide was prepared with using Cytospin (1000 rpm, 4 minutes). The slide was loaded with a mounting medium (Fluorescent Mounting Medium, S3032, Dako) and then a cover glass. Fluorescent intensity was measured with an inverted research microscope (Power IX 71, Olympus) under the conditions of exposure times of 20 ms, 50 ms, 100 ms, 200 ms and 400 ms.
Images of seven different beads (7 Peaks Beads (Cyto-Cal Multifluor Fluorescence Intensity Calibrator, FC3M, Thermo Scientific)) which respectively have different known fluorescent intensities were taken under the same conditions as above and calibration curves at respective exposure times were generated. The fluorescent signals obtained from the treated samples were converted into numbers based on the calibration curves. The obtained results are shown in Fig. 1 (Nos. 4 and 5).

### Example 2

Treated samples were prepared as illustrated in Example 1 except that Nikkol BO-20V (Nippon Chemicals Sales Co., Ltd.) was used as the nonionic surfactant instead of Emulgen 2025G. Slides were prepared from the treated samples in a similar manner as Example 1, fluorescent intensities were measured for these slides under the same conditions as Example 1 and fluorescent signals were converted into numbers. The obtained results are shown in Fig. 1 (No. 6).

### Example 3

Treated samples were prepared as illustrated in Example 1 except that Emulgen 2020G (Kao Corporation) was used as the nonionic surfactant instead of Emulgen 2025G. Slides were prepared from the treated samples in a similar manner as Example 1, fluorescent intensities were measured for the slides under the same conditions as Example 1 and fluorescent signals were converted into numbers. The obtained results are shown in Fig. 1 (No. 7). The chemical structure of Emulgen 2020G is shown in the following formula (III). 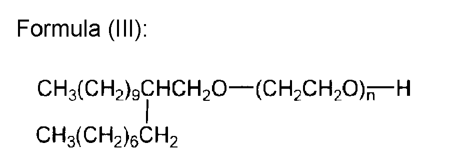 wherein n is 20.

### Comparative Example 1

In order to study an effect of mononuclear cells on the detection, conventional lysis treatment with ammonium chloride and a fixing agent was carried out instead of the treatment with the fixing agent and the nonionic surfactant in Example 1.
To the proliferated cells obtained as described in <Preparation of mononuclear cells> and <Virus proliferation> was added a 1% ammonium chloride solution in a 30-fold volume of the cells. This suspension was left to stand at room temperature for 20 minutes. To this was added 2% PFA and the mixture was left to stand at room temperature for 20 minutes to obtain a treated sample.
The obtained treated sample was centrifuged at 1500 rpm for 5 minutes with weak break, a supernatant was removed and a precipitate was obtained. The obtained precipitate was suspended in 1 ml PBS (-). The obtained suspension was centrifuged at 1500 rpm for 5 minutes with weak break, a supernatant was removed and a slide was prepared with using Cytospin (1000 rpm, 4 minutes). The slide was loaded with a mounting medium (Fluorescent Mounting Medium, S3032, Dako) and then a cover glass. Fluorescent intensities were measured under the same conditions as Example 1 and the fluorescent signals were converted into numbers.

Breast cancer cell line (MB468) was used as a positive control. The oncolytic virus was proliferated in MB468 cells in a similar manner as described in Example 1. Fluorescent intensities of the obtained MB468 cells were measured with the fluorescence microscope under the same conditions as described in Example 1 without treatment with the fixing agent and the nonionic surfactant.
The results are shown in Fig. 1. In this figure, the results of the positive control (No. 1), Comparative Example 1 (Nos. 2 and 3), Example 1 (Nos. 4 and 5), Example 2 (No. 6) and Example 3 (No. 7) are shown.

According to Fig. 1, it is found that mononuclear cells could not be removed by the method of Comparative Example 1 (Nos. 2 and 3), which showed the fluorescent signal as intense as cancer cells. On the other hand, the present methods (Nos. 4 to 7) could reduce the intense fluorescent signal derived from mononuclear cells.

It is also found that when higher alcohol-ethylene oxide adducts having a branched alkyl group-containing higher alcohol, Emulgen 2025G and Emulgen 2020G, were used as nonionic surfactants (Nos. 4, 5 and 7), the signal derived from mononuclear cells could be more efficiently reduced than the higher alcohol-ethylene oxide adduct having a linear alkyl group-containing higher alcohol, Nikkol BO-20V (No. 6).

### 2. Detection of cancer cells in blood samples

Detection of cancer cells in blood samples were practically studied with using various surfactants.

### Example 4

To 7.5 ml of peripheral blood taken from healthy human subjects was added 1120 I of anti-coagulating preservative solution (CPD solution; containing 2.63 g trisodium citrate 2H₂O, 0.327 g citric acid 2H₂O, 2.32 g D(+)-glucose and 0.251 g sodium dihydrogen phosphate 2H₂O in 100 ml sterile water) and the mixture was thoroughly mixed. The mixture was centrifuged at 1500 rpm for 5 minutes with weak break, and a supernatant (serum) was removed as much as possible with a pipette. PBS (-) was added to 14 ml and suspended. The above procedures of centrifugation and removal of serum were repeated four times. Breast cancer cell line (MB468) was added thereto to 3 x 10⁵ cells/ml to obtain a cancer cell containing blood sample.

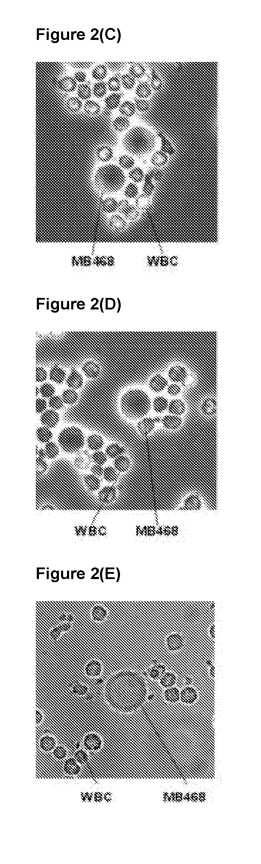

The obtained sample was treated as described in <Virus proliferation>, <Treatment with fixing agent and nonionic surfactant> and <Slide preparation and microscopy> in Example 1 and micrographed. The obtained image is shown in Fig. 2(A).

The cancer cell containing blood sample was treated as described in <Virus proliferation>, <Treatment with fixing agent and nonionic surfactant> and <Slide preparation and microscopy> in Example 1 except that Nikkol BL-9EX (nonionic surfactant, Nippon Chemicals Sales Co., Ltd.), Nikkol BO-20V (Nippon Chemicals Sales Co., Ltd.), Nikkol BT-12 (nonionic surfactant, Nippon Chemicals Sales Co., Ltd.), Emulgen 2020G (Kao Corporation), Nissan Cation AB600 (cationic surfactant, NOF Corporation) or Nissan Cation BB (cationic surfactant, NOF Corporation) was used instead of Emulgen 2025G to obtain micrographs.
Images taken with using Nikkol BL-9EX, Nikkol BO-20V, Nikkol BT-12, Emulgen 2020G, Nissan Cation AB600 and Nissan Cation BB are shown in Figs. 2(B) to (G), respectively.

### Comparative Example 2

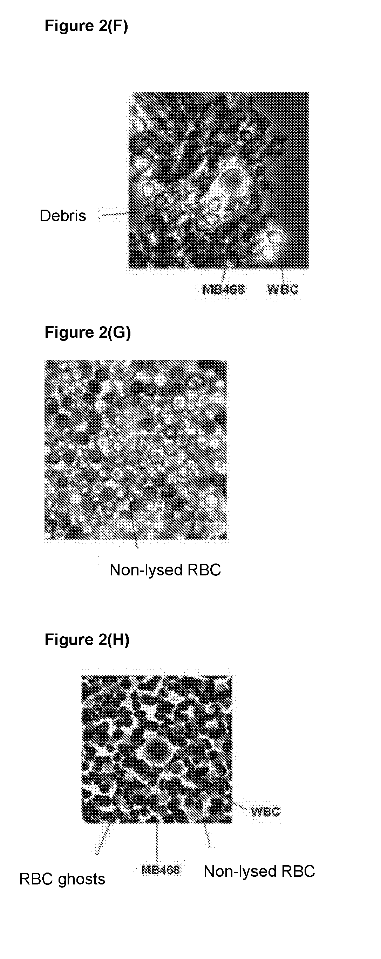

The cancer cell containing blood sample obtained as described in Example 4 was treated as described in <Virus proliferation> in Example 1 to proliferate the virus and obtain proliferated cells. The cells were suspended in an equivalent volume of 4% PFA and left to stand at room temperature for 20 minutes. This suspension was added with a 1% ammonium chloride solution in a two-fold volume of the total volume of the proliferated cells and PFA and further suspended. The suspension was incubated at 40°C for 5 minutes with stirring to obtain a treated sample.
In a similar manner as <Slide preparation and microscopy> in Example 1, the treated sample was micrographed, which is shown in Fig. 2 (H).

According to Fig. 2(H), it is shown that, for the blood sample treated by the method of Comparative Example 2, non-lysed erythrocytes (non-lysed RBCs) and debris of erythrocytes (RBC ghosts) are densely packed around the cancer cells. According to Figs. 2(F) and (G), it is shown that, for the blood samples treated by the method using cationic surfactants, debris derived from non-lysed RBCs and RBC ghosts are densely packed around the cancer cells as similar to the result of Comparative Example 2.
On the other hand, according to Figs. 2(A) to (E), it is found that, for the blood samples treated by the present method using nonionic surfactants, most of erythrocytes were removed and cancer cells are more clearly detected.

The present application relates to Japanese Patent Application No. 2008-322439 filed on December 18, 2008, whose claims, specification, drawings and abstract are entirely incorporated herein by reference.

## Claims

1. A method for detecting cancer cells in a blood sample comprising the steps of:
proliferating an oncolytic virus at least in the cancer cells by incubating the blood sample being suspected to contain the cancer cells with the oncolytic virus;
mixing the blood sample obtained from the proliferating step with a fixing agent and a nonionic surfactant; and
detecting the cancer cells in the blood sample obtained from the mixing step, in which the oncolytic virus has been proliferated.

2. The method according to claim 1, wherein, in the mixing step, the blood sample is mixed with the fixing agent followed by mixing of the nonionic surfactant.

3. The method according to claim 1, wherein, in the mixing step, a concentration of the nonionic surfactant when it is mixed with the blood sample is such that erythrocytes and leukocytes are damaged without damaging the cancer cells.

4. The method according to claim 1, wherein the nonionic surfactant is a polyoxyethylene nonionic surfactant.

5. The method according to claim 4, wherein the polyoxyethylene nonionic surfactant is a higher alcohol-ethylene oxide adduct.

6. The method according to claim 5, wherein an alkyl group of the higher alcohol in the higher alcohol-ethylene oxide adduct is branched.

7. The method according to claim 1, wherein, in the mixing step, a concentration of the fixing agent when it is mixed with the blood sample is such that erythrocytes are lysed.

8. The method according to claim 1, wherein the fixing agent is paraformaldehyde, glutaraldehyde or formaldehyde.

9. The method according to claim 1, wherein the oncolytic virus expresses a label protein.

10. The method according to claim 1, wherein the oncolytic virus is incorporated with a telomerase promoter.

11. The method according to claim 9, wherein, in the detecting step, a signal from the label protein is detected.

12. A reagent kit for detection of cancer cells in a blood sample comprising a first reagent containing an oncolytic virus;
a second reagent containing a fixing agent; and
a third reagent containing a nonionic surfactant.

13. The kit according to claim 12, wherein the third reagent comprises the nonionic surfactant in an amount such that erythrocytes and leukocytes are damaged without damaging the cancer cells when it is mixed with the blood sample.

14. The kit according to claim 12, wherein the nonionic surfactant is a polyoxyethylene nonionic surfactant.

15. The kit according to claim 14, wherein the polyoxyethylene nonionic surfactant is a higher alcohol-ethylene oxide adduct.

16. The kit according to claim 15, wherein an alkyl group of the higher alcohol in the higher alcohol-ethylene oxide adduct is branched.

17. The kit according to claim 12, wherein the second reagent comprises the fixing agent in an amount such that erythrocytes are lysed when it is mixed with the blood sample.

18. The kit according to claim 12, wherein the fixing agent is paraformaldehyde, glutaraldehyde or formaldehyde.

19. The kit according to claim 12, wherein the oncolytic virus expresses a label protein.

20. The kit according to claim 12, wherein the oncolytic virus is incorporated with a telomerase promoter.
